# EUROPEAN PATENT APPLICATION

(11) **EP 4 310 186 A1**
(43) Date of publication of application: **24.01.2024**
(21) Application number: 21931645.2
(22) Date of filing: 10.05.2021
(51) Int. Cl.: C12N 15/45, A61K 39/165, A61K 39/215, A61K 48/00, A61P 37/04, C12N 15/50, C12N 15/86

(54) **RECOMBINANT MEASLES VIRUS**

(30) Priority: 15.03.2021 JP 2021041015
(71) Applicant: Yoneda, Misako, Tokyo 134-0085 (JP); Kai, Chieko, Tokyo 153-0041 (JP)
(72) Inventor: Yoneda, Misako, Tokyo 134-0085 (JP); Kai, Chieko, Tokyo 153-0041 (JP)
(74) Representative: De Vries & Metman
(86) International application number: PCT/JP2021/017632
(87) International publication number: WO 2022/195900

(57) **Abstract**

The present invention provides a recombinant measles virus useful as a live vaccine against COVID-19 and a vector used for production of the recombinant measles virus. That is, the present invention relates to a recombinant measles virus having a gene encoding a protein of the coronavirus SARS-CoV-2 inserted between the N gene region and the P gene region in a measles virus genome; the recombinant measles virus in which the protein is a spike protein of SARS-CoV-2 or a partial protein thereof; and a DNA in which a gene encoding a protein of SARS-CoV-2 is inserted in a region ranging from the 1 ,686th base to the 1 ,694th base of a base sequence set forth in SEQ ID NO: 2.

## Description

### [Technical Field]

The present invention relates to a recombinant measles virus useful as a vaccine against a novel coronavirus infectious disease (COVID-19).

Priority is claimed on Japanese Patent Application No. 2021-041015, filed March 15, 2021, the content of which is incorporated herein by reference.

### [Background Art]

COVID-19 is an infectious disease caused by a novel coronavirus, Severe Acute Respiratory Syndrome Coronavirus 2 (SARS-CoV-2), as a causative pathogen. Coronaviruses invade host cells by a spike glycoprotein interacting with an angiotensin-converting enzyme of a receptor on the host cell surface.

COVID-19 has caused a pandemic and has become a global threat. Since the first infection was reported in December 2019, the number of infected individuals and the number of deaths have been increasing, and there is no sign that the number of cases may decrease. To end this pandemic, the development of therapeutic drugs or vaccines is of utmost importance, and there is a high demand for development of excellent vaccines worldwide. In addition, vaccines developed in each country are preferentially supplied to their own nation, and therefore, there is a need for Japan to develop its own vaccines.

Vaccines are broadly classified into live vaccines and inactivated vaccines. Many of the vaccines against COVID-19 which are currently being developed in various countries include the so-called inactivated vaccines, such as an inactivated virus, a SARS-CoV-2 protein, and a nucleic acid (mRNA and DNA) for expressing the protein in a human body, and are mainly antigen-induced vaccines that induce humoral immunity. These inactivated vaccines have a short duration of immunity and are required to be administered frequently, and a large amount of preparations thereof need to be prepared. In addition, in COVID-19, antibody-dependent enhancement (ADE) is suggested to occur, and there is also a concern about the increasing severity of side effects caused by a large amount of antibodies being induced by frequent administration.

On the other hand, measles is caused by measles viruses classified under the family Paramyxoviridae, genus Morbillivirus. A hematoaggregin (H) protein and a fusion (F) protein are present in the external envelope of a measles virus, while in the internal capsid, a nucleocapsid (N) protein, a phosphoprotein (P), a large (L) protein, and a matrix (M) protein are present along with a helical negative RNA genome. The measles virus genome has, at both ends, a leader sequence and a trailer sequence involved in the replication of the virus, and genes encoding N, P, M, F, H, and L proteins are present in order from the 3' end, between the leader sequence and the trailer sequence. From the P gene region, not only the P protein but also the V protein and the C protein are translated.

Measles is an acute systemic infectious disease and may cause serious diseases such as pneumonia and encephalitis. The measles virus is highly infectious and infects through various routes such as airborne infection, droplet infection, and contact infection. For measles, there is no fundamental therapeutic drug, and vaccination is the most effective prophylactic method. As a vaccine against the measles, a live attenuated vaccine obtained by passaging a measles virus in a cell culture to weaken its virulence is used.

The live measles vaccine has excellent characteristics such as induction of strong cellular immunity and exhibition of extremely long immunity persistence referred to as permanent immunity. Furthermore, the live measles vaccine has been used all over the world for 50 years or longer, and its safety in humans has also been confirmed. Therefore, live vaccines against other diseases have been developed by using a genetic recombinant virus vector in which genes of microorganisms causing other diseases and the like are inserted into a genomic RNA of an attenuated measles strain virus useful as the measles vaccine. For example, a recombinant measles virus obtained by inserting an F gene or a G gene encoding a membrane protein of a Nipah virus into a measles virus genome functions as a bivalent vaccine against both measles and Nipah virus infectious disease (Patent Document 1). In addition, a recombinant measles virus obtained by inserting a gene of a protein of a malaria parasite into a measles virus genome functions as a bivalent vaccine against both measles and malaria infectious disease (Patent Document 2).

### [Citation List]

### [Patent Documents]

[Patent Document 1]
   Japanese Unexamined Patent Application, First Publication No. 2010-115154
[Patent Document 2]
   Japanese Unexamined Patent Application, First Publication No. 2010-099041

### [Non-Patent Document]

[Non-Patent Document 1]
Rota et al, Virus Research, 1994, vol. 31 (3), p. 317-330.

### [Summary of Invention]

### [Technical Problem]

A main object of the present invention is to provide a recombinant measles virus useful as a live vaccine against COVID-19, and a vector used for production of the recombinant measles virus.

### [Solution to Problem]

The present inventors have conducted extensive studies, and as a result, they have found that a recombinant measles virus in which a gene encoding a spike protein of SARS-CoV-2 or a partial protein thereof is inserted into a cDNA of a measles virus genome is useful as a live vaccine against COVID-19, leading to completion of the present invention.

The recombinant measles virus and the like according to the present invention are as follows.
[1] A recombinant measles virus, including a gene encoding a protein of coronavirus SARS-CoV-2 or a partial protein thereof inserted between an N gene region and a P gene region in a measles virus genome.
[2] The recombinant measles virus according to [1],
   in which the protein is a spike protein of SARS-CoV-2 or a partial protein thereof.
[3] The recombinant measles virus according to [1] or [2],
   in which the measles virus genome is an RNA consisting of a base sequence complementary to a base sequence set forth in SEQ ID NO: 1.
[4] A genomic RNA of the recombinant measles virus according to any one of [1] to [3].
[5] An RNA consisting of a base sequence complementary to a base sequence set forth in SEQ ID NO: 3.
[6] A DNA including a gene encoding a protein of SARS-CoV-2 inserted into a region ranging from a 1,686th base to a 1,694th base in a base sequence set forth in SEQ ID NO: 2.
[7] A DNA including a gene encoding a protein of SARS-CoV-2 or a partial protein thereof inserted into an Fse I recognition sequence located from a 1,686th base to a 1,694th base of a base sequence set forth in SEQ ID NO: 1.
[8] A DNA consisting of a base sequence set forth in SEQ ID NO: 3.
[9] A vector for producing a COVID-19 vaccine, the vector including the DNA according to any one of [6] to [8].
[10] A plasmid vector including a DNA consisting of a base sequence set forth in SEQ ID NO: 1.
[11] A method for producing a recombinant measles virus, the method including transfecting a vector for producing a COVID-19 vaccine including the DNA according to any one of [6] to [8] and an expression vector for an N protein, a P protein, and an L protein of a measles virus into a cell expressing an RNA polymerase capable of transcribing an RNA using the DNA as a template to produce a recombinant measles virus in the cell.
[12] A pharmaceutical composition including:
   the recombinant measles virus according to any one of [1] to [3]; and
   a pharmaceutically acceptable carrier.
[13] The pharmaceutical composition according to [12],
   in which the pharmaceutical composition is used as a bivalent vaccine against measles and COV1D-19.
[14] A use of the recombinant measles virus according to any one of [1] to [3] as a vaccine against COVID-19.
[15] A use of the recombinant measles virus according to any one of [1] to [3] for producing a vaccine against COVID-19.
[16] A method for inducing an immune response against coronavirus SARS-CoV-2, the method including:
   administering a pharmaceutical composition including the recombinant measles virus according to any one of [1] to [3] to a subject in need of prevention of COVID-19.

### [Advantageous Effects of Invention]

The recombinant measles virus according to the present invention is useful as a vaccine against COV1D-19.

In addition, the recombinant measles virus according to the present invention can be produced by a vector for producing a COVID-19 vaccine according to the present invention and the method for producing a recombinant measles virus using the vector.

### [Brief Description of Drawings]

FIG. 1 is a view schematically showing the genomic structure of a recombinant measles virus (MV-CoVS) having a spike protein (CoV-S) gene of SARS-CoV-2 in Example 1.
FIG. 2 shows a transmission image of a Vero cell in which CPE was observed (left figure) and an image obtained by fluorescent immunostaining of a spike protein of SARS-CoV-2 (right figure) in Example 1.
FIG. 3 is a view showing the results of Western blotting analysis using an antibody (anti-MV-N antibody) recognizing an N protein of a measles virus and an anti-CoV-S antibody with regard to a Vero cell (non-infected cell) not infected with a measles virus, a Vero cell (MV-Ed cell) infected with a measles virus MV-Ed, and a Vero cell (MV-CoVS cell) infected with a measles virus MV-CoVS in Example 1.
FIG. 4 is a view showing the measurement results for a titer of an anti-CoV-S antibody in the serum over time by inoculating hamsters twice with each of the measles virus MV-CoVS and the measles virus MV-Ed in Example 1.

### [Description of Embodiments]

In the present invention and the present specification, the term "measles virus genome" means a genomic RNA capable of producing an infectious measles virus, the genomic RNA having no foreign gene encoding a protein. The measles virus genome may be either a genomic RNA of wild-type epidemic measles virus or a genomic RNA of an attenuated measles strain, or may be an RNA obtained by subjecting the genomic RNA to various genetic modification treatments other than insertion of a foreign gene encoding a protein.

In the present invention and the present specification, the term "recombinant measles virus" is an infectious virus which expresses a foreign protein. In the genomic RNA of the recombinant measles virus, a foreign structural gene encoding a protein is inserted into a region other than a gene region encoding a protein of measles virus.

In the recombinant measles virus according to the present invention, a gene encoding a protein of coronavirus SARS-CoV-2 or a partial protein thereof is inserted between the N gene region and the P gene region in the measles virus genome. In a recombinant measles virus in which a protein of SARS-CoV-2 or a partial protein thereof is inserted between the N gene region and the P gene region of the measles virus genome, the protein of SARS-CoV-2 or a partial protein is expressed within the infected cell to induce immunity against these proteins. In addition, since the recombinant measles virus further maintains a function as a measles virus, it is also effective as a vaccine against measles.

The recombinant measles virus according to the present invention induces the production of an antibody against the protein of SARS-CoV-2 or a partial protein thereof expressed in the infected cell. Therefore, the recombinant measles virus induces the production of an antibody against SARS-CoV-2 and has a strong protective effect against COVID-19 and measles. Furthermore, similar to the live measles vaccine used in the related art, the recombinant measles virus can be expected to induce not only humoral immunity but also cellular immunity, and to confer immunity persistence for a long period of time called permanent immunity. In addition, the likelihood of ADE occurring through the administration of the recombinant measles virus is expected to be low. Similar to the live measles vaccines in the related art, the recombinant measles virus makes it possible to produce a pharmaceutical composition at a comparatively low cost and to make it into a freeze-dried preparation, whereby the problem of cold chain storage can also be solved.

For the production of the recombinant measles virus according to the present invention, for example, a reverse genetic system used for the reconstruction of a canine distemper virus or a measles virus described in Japanese Unexamined Patent Application, First Publication No. 2001-275684 or Japanese Unexamined Patent Application, First Publication No. 2010-115154 can be used. The measles virus belongs to a (-) strand RNA virus, and modification of the viral genome and introduction of a foreign gene can be performed using a widely used genetic modification technique for a cDNA of the measles virus genome. In the reverse genetic system, recombinant measles virus particles having infectivity are produced from a recombinant cDNA obtained by introducing a foreign gene into a cDNA of a genomic RNA of the measles virus. The genomic RNA of the produced recombinant measles virus is an RNA consisting of a base sequence complementary to the recombinant cDNA used for the production.

### <Recombinant cDNA>

Specifically, for the cDNA of the measles virus genome, first, a gene encoding a protein of SARS-CoV-2 or a partial protein thereof is incorporated between the N gene region and the P gene region to obtain a recombinant cDNA.

The measles virus genome used for production of the recombinant measles virus according to the present invention is not particularly limited as long as it is a genomic RNA of a measles virus capable of inducing a neutralizing antibody against wild-type epidemic measles virus. The measles virus may be, for example, a natural virus strain such as an HL strain and an ICB strain, or an attenuated strain such as an Edmonston strain and an AIC strain, or may be a genetically modified virus strain within a range not impairing induction for producing a neutralizing antibody against the measles virus.

As the recombinant measles virus according to the present invention, a recombinant measles virus having a protein of SARS-CoV-2 or a partial protein thereof incorporated into a genomic RNA of an attenuated strain, or a genetically modified virus strain thereof is preferable from the viewpoint that it can be expected to have a higher protective effect against COVID-19 and higher safety.

Examples of the attenuated measles strain include a strain obtained by passaging an Edmonston wild-type strain to a measles non-susceptible animal-derived cell. The sequence identity of full-length genomic RNAs and the number of mismatched bases of various known attenuated strains are shown in Table 1 (Non-Patent Document 1).

**[Table 1]**

| Strain | Number of mismatched bases | Identity |
|---|---|---|
| Leningrad-4 | 36 | 99% |
| Edmonston B | 38 | 99% |
| Edmonston wt | 42 | 99% |
| Zagreb | 42 | 99% |
| Moraten | 44 | 99% |
| Schwarz | 44 | 99% |
| Changchun-47 | 47 | 99% |
| AIK-C | 48 | 99% |
| Shanghai-191 | 56 | 99% |
| CAM-70 | 75 | 99% |

The strain is affected by the characteristics of a recombinant measles virus thus obtained, depending on the type of attenuated strain used as a host. As the attenuated measles strain used as a host for constructing the recombinant measles virus according to the present invention, an attenuated measles strain having, as a genomic RNA, an RNA consisting of a base sequence complementary to a base sequence set forth in SEQ ID NO: 2 is preferable. The genomic RNA has 30 bases different from the full-length genomic RNA (GenBank Accession No.: Z66517) of the Edmonston B strain (Rubeovax).

As the cDNA of the measles virus genome used in constructing the recombinant measles virus according to the present invention, a DNA in which a restriction enzyme recognition sequence for inserting a foreign structural gene is arranged as appropriate between each of N, P, M, F, H, and L genes encoding proteins constituting the viruses. In particular, it is preferable to use a cDNA of a measles virus genome in which a restriction enzyme recognition sequence is positioned between the N gene region and the P gene region (which may be hereinafter referred to as a "modified cDNA").

The restriction enzyme recognition sequence inserted between the N gene region and the P gene region of the cDNA of the measles virus genome is not particularly limited as long as it is a restriction enzyme recognition sequence that can exist between the N gene region and the P gene region at only one site in the entire length of the cDNA. Specifically, for example, a recognition sequence such as Fse I can be used. The insertion of a restriction enzyme recognition sequence between any of the genes encoding proteins constituting a virus in the cDNA of the measles virus genome can be carried out by, for example, the methods described in Patent Document 1 and Patent Document 2.

As the modified cDNA used for constructing the recombinant measles virus according to the present invention, a DNA consisting of a base sequence in which a restriction enzyme recognition sequence is positioned between the N gene region and the P gene region in the base sequence set forth in SEQ ID NO: 2 is preferable, a DNA consisting of a base sequence in which an Fse I recognition sequence is inserted between the N gene region and the P gene region in the base sequence set forth in SEQ ID NO: 2 is more preferable, and a DNA consisting of the base sequence (GenBank Accession No.: MT409882) set forth in SEQ ID NO: 1 is still more preferable. Furthermore, the base sequence set forth in SEQ ID NO: 1 is a base sequence having an Fse I recognition sequence in the region ranging from the 1 ,686th base to the 1 ,694th base between the N gene region and the P gene region in the base sequence set forth in SEQ ID NO: 2.

The protein of SARS-CoV-2 or a partial protein thereof may be a protein present in SARS-CoV-2 or a partial protein thereof. The recombinant measles virus according to the present invention is preferably the spike protein of SARS-CoV-2 or a partial protein thereof from the viewpoint that this can be expected to have a higher protective effect against COVID-19. The cDNA of a gene encoding the spike protein of SARS-CoV-2 or a partial protein thereof can be obtained by an ordinary method based on a base sequence of a genomic RNA of SARS-CoV-2 (NCBI Accession No.: NC_045512.2). Furthermore, the gene region encoding the spike protein of SARS-CoV-2 is a region ranging from the 21,563rd base to the 25,384th base of the genomic RNA.

For example, by performing RT-PCR using a genomic RNA extracted from SARS-CoV-2 and a primer designed based on the base sequence information of the genomic RNA, the fragment of cDNA encoding the spike protein or a partial protein thereof can be synthesized. The primer can be designed using software generally used for designing a PCR primer. In a case where a fragment of the cDNA encoding the spike protein or a partial protein thereof is inserted into a modified cDNA in which a restriction enzyme recognition sequence is positioned between the N gene region and the P gene region, RT-PCR is performed using a primer in which the restriction enzyme recognition sequence is arranged at the 5' end. This makes it possible to obtain a DNA fragment in which the restriction enzyme recognition sequence is arranged at both ends of the cDNA encoding the spike protein of a partial protein thereof. Alternatively, a full-length DNA fragment of the cDNA encoding the spike protein or a partial protein thereof, or a DNA fragment in which restriction enzyme recognition sequences are arranged at both ends of the cDNA can also be produced by chemical synthesis.

A DNA fragment encoding a protein of SARS-CoV-2 or a partial protein thereof is inserted between the N gene region and the P gene region in the cDNA of the measles virus genome to obtain a recombinant cDNA. In a case of using a restriction enzyme recognition sequence, both the DNA fragment of the modified cDNA in which the restriction enzyme recognition sequence is located between the N gene region and the P gene region, and the DNA fragment having the restriction enzyme recognition sequence arranged at both ends of the cDNA encoding the protein of SARS-CoV-2 or a partial protein thereof are subjected to a restriction enzyme treatment, and then linked to each other by a ligation reaction, whereby a desired recombinant cDNA can be obtained.

The recombinant measles virus according to the present invention may undergo modifications such as insertion, deletion, or substitution of bases at any site in the genomic RNA as long as the preventive ability against COVID-19 and measles is maintained. For example, genes involved in RNA replication of some measles virus may be modified in order to inactivate genes involved in immunogenicity of to increase the transcription efficiency or the replication efficiency of RNAs. Specific examples of the modification include a modification of an intervening sequence and a modification of a leader portion.

In the recombinant measles virus according to the present invention, any other foreign gene may be inserted into any site in the genomic RNA as long as the preventive ability against COVID-19 and measles is maintained. Examples of other foreign genes to be inserted include genes encoding various proteins that induce pathogenicity in viruses, bacteria, and parasites which can be expressed in the host, genes encoding various cytokines, genes encoding various peptide hormones, and genes encoding antigen recognition sites in the antibody molecules. Examples of such genes include genes encoding various proteins, such as influenza virus, Mumps virus, HIV, dengue virus, diphtheria, and leishmania, or antigen recognition site genes of monoclonal antibodies against cancer-specific marker molecules.

### <Vector for Producing COVID-19 Vaccine>

A vector including a DNA in which the recombinant cDNA is connected downstream of a specific promoter can be used for production of the recombinant measles virus according to the present invention. The recombinant measles virus according to the present invention is effective as a vaccine against COVID-19, and the vector is used as a vector for producing a COVID-19 vaccine. The vector for producing a COVID-19 vaccine is preferably a plasmid vector, but may be a linear DNA vector.

For example, a vector for producing a COVID-19 vaccine can be produced by connecting a DNA fragment including the promoter sequence to a DNA fragment including the recombinant cDNA such that the recombinant cDNA is connected downstream of the promoter sequence, and incorporating the linked construct into a plasmid. The linking between DNA fragments and the insertion into a plasmid can be performed by a genetic recombination technique.

In the vector for producing a COVID-19 vaccine, the promoter upstream of the recombinant cDNA can be appropriately selected from promoters recognized by known DNA-dependent RNA polymerases, and then used. Examples of the promoter include a T7 promoter, an SP6 promoter, and a T3 promoter.

Specifically, as the vector for producing a COVID-19 vaccine, a plasmid vector incorporated with a DNA having a gene encoding the protein of SARS-CoV-2 is inserted into a region ranging from the 1,686th base to the 1,694th base in the base sequence set forth in SEQ ID NO: 2, a DNA in which a gene encoding the protein of SARS-CoV-2 is inserted into an Fse I recognition site located at the 1,686th base to the 1,694th base of the base sequence set forth in SEQ ID NO: 1, or a DNA fragment in which a promoter is linked upstream of any DNA of DNAs consisting of the base sequence set forth in SEQ ID NO: 3 is preferable, and a plasmid vector incorporated with a DNA fragment in which a T7 promoter, a T3 promoter, or an SP6 promoter is linked upstream of a DNA consisting of the base sequence set forth in SEQ ID NO: 3 is more preferable.

### <Gene Recombinant Vector>

The vector for producing a COVID-19 vaccine can be more easily produced by using a plasmid vector including a modified cDNA in which a restriction enzyme recognition sequence is arranged between the N gene region and the P gene region. Among the plasmid vectors, in particular, a plasmid vector in which a modified cDNA having a restriction enzyme recognition sequence arranged between the N gene region and the P gene region is connected downstream of a promoter is useful as a gene recombinant vector for producing a bivalent vaccine against measles and other diseases.

The insertion of a modified cDNA or a promoter fragment into a plasmid vector can be performed by a genetic modification technique such as a ligation reaction. Furthermore, the promoter connected upstream of the modified cDNA can be appropriately selected from promoters recognized by known DNA-dependent RNA polymerases such as a T7 promoter, a T3 promoter, and a SP6 promoter, and then used.

As the modified cDNA to be inserted into the genetic recombinant vector, a DNA consisting of a base sequence in which a restriction enzyme recognition sequence is arranged in a region ranging from the 1,686th base to the 1,694th base in the base sequence set forth in SEQ ID NO: 2 is preferable, and a DNA consisting of the base sequence set forth in SEQ ID NO: 1 is more preferable. A plasmid vector including a base sequence in which the base sequence set forth in SEQ ID NO: 1 is connected downstream of a promoter sequence is very useful as a genetic recombinant vector.

For example, for a plasmid vector including a base sequence in which the base sequence set forth in SEQ ID NO: 1 is connected downstream of a promoter sequence, a vector for producing a COVID-19 vaccine can be produced by incorporating a DNA fragment having a base sequence in which an Fse I recognition sequence is arranged at both ends of a gene encoding a spike protein or a partial protein thereof into an Fse I recognition sequence in the base sequence set forth in SEQ ID NO: 1 by genetic manipulation. In addition, it is possible to produce a vector that enables the production of a recombinant measles virus effective as a vaccine against both measles and other diseases by incorporating a DNA fragment including a gene encoding a protein that induces an immune response effective for preventing COVID-19 and diseases other than measles, instead of a DNA fragment including a gene encoding the spike protein or a partial protein thereof.

### <Method for Producing Recombinant Measles Virus>

The vector for producing a COVID-19 vaccine is transfected into a cell which carries an RNA polymerase capable of transcribing an RNA using a cDNA of the measles virus genome incorporated into the vector as a template, and any in a group of enzymes required for the transcriptional replication of the measles virus. Inside the cell, a genomic RNA of the recombinant measles virus is synthesized using the cDNA of the measles virus genome incorporated into the vector as a template by the polymerase. Inside the cell, a recombinant measles virus is produced by the genomic RNA and the group of enzymes required for the transcriptional replication of the measles virus. That is, the genomic RNA of the produced recombinant measles virus is an RNA consisting of a base sequence complementary to the recombinant cDNA used for production.

Examples of the RNA polymerase for transcribing an RNA using cDNA of the measles virus genome as a template include a DNA-dependent RNA polymerase that acts on a promoter connected upstream of the cDNA of the measles virus genome incorporated into the vector for producing a COVID-19 vaccine. In a case where the promoter connected upstream of the cDNA of the measles virus genome is a T7 promoter, for example, a T7 RNA polymerase can be used as the DNA-dependent RNA polymerase. Examples of the cell expressing the RNA polymerase include a cell infected in advance with a recombinant vaccinia virus that expresses the T7 RNA polymerase, and a T7 RNA polymerase constant expression strain in which the T7 RNA polymerase gene is incorporated into a chromosome of a cultured cell.

Examples of the group of enzymes required for the transcriptional replication of the measles virus include the N protein, the P protein, and the L protein of the measles virus. Examples of the cell carrying all the group of enzymes required for the transcriptional replication of the measles virus include a cell transfected with all of an expression vector incorporated with a gene encoding the N protein (an expression vector for the N protein), an expression vector incorporated with a gene encoding the P protein (an expression vector for the P protein), and an expression vector incorporated with a gene encoding the L protein (an expression vector for the L protein).

For example, any of the vector for producing a COVID-19 vaccine, the expression vector for the N protein, the expression vector for the P protein, and the expression vector for the L protein are transfected into the cultured cell infected in advance with the recombinant vaccinia virus that expresses a T7 RNA polymerase are transfected together. Inside the transfected cell, a recombinant measles virus carrying a genome with an RNA consisting of a base sequence complementary to the recombinant cDNA used for production is constructed.

The produced recombinant measles virus can be used as a vaccine against COVID-19. That is, the produced recombinant measles virus proliferates in the infected cells, and further, the spike protein of SARS-CoV-2 or a partial protein thereof is expressed to induce the production of an antibody against the protein. Therefore, the recombinant measles virus functions as a vaccine against COVID-19 as well as a vaccine against measles.

### <Pharmaceutical Composition>

A vaccine against COV1D-19 can be produced by using the recombinant measles virus according to the present invention. For example, a pharmaceutical composition including the recombinant measles virus and the pharmaceutically acceptable carrier in which the pharmaceutical composition is useful as a bivalent vaccine against measles and COVID-19 can be produced by appropriately mixing the recombinant measles virus according to the present invention and a pharmaceutically acceptable carrier. The production of the pharmaceutical composition including the recombinant measles virus and the pharmaceutically acceptable carrier can be carried out using appropriate additives as necessary by a method which is commonly used in the field of production of pharmaceuticals.

The pharmaceutically acceptable carrier is a diluent, an excipient, a binder, a solvent, or the like which does not cause a harmful physiological reaction to an administration subject and does not result in a harmful interaction with other ingredients such as pharmacologically active ingredients. As the carrier, specifically, water, physiological saline, various buffer solutions, or the like is used. In addition, examples of the additives that can be used include an adjuvant, a diluent, an excipient, a binder, a stabilizer, an isotonic agent, a buffering agent, a solubilizing agent, a suspending agent, a preservative, an antifreeze agent, a cryoprotective agent, a freeze-drying protective agent, and a bactericidal agent.

An adjuvant commonly used in the field of pharmaceuticals can be used as the adjuvant for enhancing immunogenicity. Specific examples thereof include microbial cells such as BCG and Propionibacterium acnes, cell walls, microbial cell components such as trehalose dimycolate (TDM), synthetic compounds such as lipopolysaccharides (LPS) or lipid A fractions of which are endotoxins of Gram-negative bacteria, β-glucan, N-acetylmuramyl dipeptide (MDP) pestatin, or levamiazole, proteins derived from biological components such as thymus hormones, thymus factors, and tuftsin, peptide substances, Freund's incomplete adjuvant, and Freund's complete adjuvant.

The dosage form of the vaccine may be any one of a liquid form, a frozen product, and a freeze-dried product. A culture solution obtained by culturing in an appropriate medium, cultured cells, and the like is collected, an additive such as a stabilizer is added thereto, and the mixture is dispensed into a small bottle, an ampoule, or the like, and then sealed, whereby a liquid vaccine can be produced. After being dispensed, the vaccine that is frozen by gradually lowering the temperature is a frozen vaccine, to which an antifreeze agent or a freeze-protective agent is added. In a case where the dispensed container is frozen in a freeze dryer and then vacuum-dried, and the container is sealed as is or by being filled with nitrogen gas, it is possible to obtain a freeze-dried vaccine. The liquid vaccine is used as is or after being diluted with physiological saline or the like. For the frozen vaccine and the dried vaccine, a dissolution solution for dissolving the vaccine at the time of use is used. The solutions for dissolution are various buffer solutions or physiological saline.

An immune response against SARS-CoV-2 can be induced in the subject by administering an effective amount of a pharmaceutical composition including the recombinant measles virus according to the present invention to a subject as a vaccine. The administration of the vaccine can be performed by subcutaneous administration, intramuscular administration, intravenous administration, or the like. The dosage varies depending on age, body weight, sex, administration method, or the like of the subject and is not particularly limited, but is preferably in a range of usually 10⁴ to 10⁷ TCID₅₀ per dose, and particularly preferably at least 10⁴ TCID₅₀. The administration is preferably performed in the same manner as that for the measles vaccine.

In addition, after various animals including humans are infected with the recombinant measles virus according to the present invention, antisera and the like can be obtained from body fluids collected from animals for use in treatment or diagnosis.

### [Examples]

Next, the present invention will be more specifically described with reference to Examples and the like, but the present invention is not limited to Examples.

### [Example 1]

A recombinant measles virus (MV-CoVS) having a spike protein (CoV-S) gene of SARS-CoV-2 was constructed.

### <Preparation of Plasmid pMV-CoVS Including cDNA of Genomic RNA of Recombinant Measles Virus Having CoV-S Gene>

First, an RNA was extracted from a measles virus that is an attenuated measles strain with a genomic RNA in which the RNA consists of a base sequence complementary to the base sequence set forth in SEQ ID NO: 2, and RT-PCR was performed using the obtained RNA as a template. The full-length genomic RNA was divided into several partial regions and subjected to RT-PCR, and the obtained amplified fragments were linked to obtain a plasmid pMV-Ed in which a DNA fragment consisting of the base sequence set forth in SEQ ID NO: 1 was linked downstream of the T7 promoter in the plasmid pMDB1.

The cDNA of the CoV-S gene was obtained by RT-PCR using the entire RNA extracted from the SARS-CoV-2-infected Vero cell. PCR was carried out using the obtained cDNA as a template and using a primer set having an Fse 1 recognition sequence at the 5' end, thereby obtaining a DNA fragment encoding the spike protein having Fse I recognition sequences at both ends (CoV-S fragment: SEQ ID NO: 4).

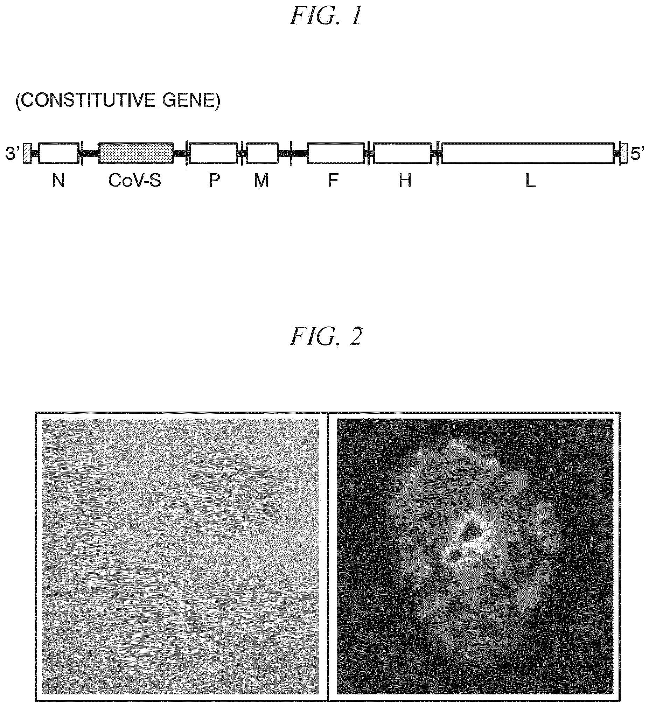

The plasmid pMV-Ed and the CoV-S fragment were digested with Fse 1, and the two were linked by ligation. The product was inserted into the Fse I recognition site of pMV-Ed to obtain a plasmid pMV-CoVS having a cDNA having the genomic structure shown in FIG. 1.

### <Reconstitution of MV-CoVS>

For the replication of the measles virus, an N protein expression vector pGEM-NP in which the N protein gene of the measles virus was inserted into a plasmid pGEM, a P protein expression vector pGEM-P in which the P protein gene of the measles virus was inserted into pGEM, and L protein expression vectors pGEM-L and pMV-CoVS in which the L protein gene of the measles virus was inserted into pGEM were used. 2 µg of pGEM-NP, 2 µg of pGEM-P, 0.2 µg of pGEM-L, and 2 µg of pMV-CoVS were added into a 1.5 mL sampling tube containing 1 mL of Opti-MEM (manufactured by Thermo Fisher Scientific, Inc.) to prepare a nucleic acid solution. 1 mL of Opti-MEM was collected into another sampling tube, a transfection reagent "Lipofectamin LTX" (manufactured by Thermo Fisher Scientific, Inc.) was added thereto and mixed, and then the mixture was left to stand at room temperature for 5 minutes in this state. Thereafter, the transfection reagent solution was mixed with the nucleic acid solution, and the mixture was further allowed to stand at room temperature for 15 minutes or longer and used as a plasmid solution.

Cultured cell strain BHK-T7 cells which had been subjected to a common trypsin treatment were added into each well of a 6-well plate at a density of 5 × 10⁵ cells per well along with 2 mL of a culture medium (a DMEM medium including 2% fetal bovine serum), and the plate was cultured for 6 hours under conditions of 5% CO₂ and 37°C. Thereafter, 200 µL of the plasmid solution was added dropwise to each well.

The cells in the 6-well plate were cultured for 2 days under the conditions of 5% CO₂ and 37°C. After removing the culture medium on the second day, the Vero cells suspended in VP-SFM (manufactured by Gibco BRL/Life Technologies) were seeded at a density of 5 × 10⁵ cells per well, and the cells were overlaid on the BHK-T7 cells. In a case where the plate was further cultured under the conditions of 5% CO₂ and 37°C, a cytopathic effect (CPE) was observed on the Vero cells. The CPE thereof indicates that MV-CoVS increased in the Vero cells infected with the recombinant measles virus MV-CoVS reconstituted in BHK-T7 cells.

RT-PCR was performed using, as a template, the RNA extracted from the Vero cells in which CPE was observed, and sequence analysis of the obtained PCR fragment was performed to confirm that the recombinant measles virus MV-CoVS was reconstructed in the Vero cells.

Furthermore, the Vero cells in which CPE was observed were subjected to fluorescent immunostaining using an antibody against CoV-S (anti-CoV-S antibody) as a primary antibody. First, the Vero cells were infected with the recombinant measles virus MV-CoVS, and after 48 hours, the cells were fixed with 4% paraformaldehyde and permeabilized with 0.2% Triton X-100. Next, a 1,000-fold diluted anti-CoV-S antibody (mouse serum) was added thereto and allowed to react at room temperature for 1 hour. After removing the anti-CoV-S antibody and washing the cells three times with PBS, an Alexa 488-labeled anti-mouse IgG antibody was diluted 500-fold and added thereto, and incubation was performed at room temperature for 30 minutes to cause a reaction. After removing the Alexa488-labeled anti-mouse IgG antibody and washing the cells five times with PBS, the MV-CoVS-infected Vero cells were then observed using a confocal laser scanning microscope.

A transmission image of MV-CoVS-infected Vero cells (left figure) and an image obtained by fluorescent immunostaining of a spike protein of SARS-CoV-2 (right figure) are shown in FIG. 2. As shown in FIG. 2, Alexa488 fluorescence was not observed in the Vero cells not infected with MV-CoVS, but Alexa488 fluorescence was observed in the MV-CoVS-infected Vero cells. From these results, it was confirmed that the CoV-S protein was expressed in the Vero cells infected with the recombinant measles virus MV-CoVS, that is, that a recombinant measles virus expressing the CoV-S protein could be constructed.

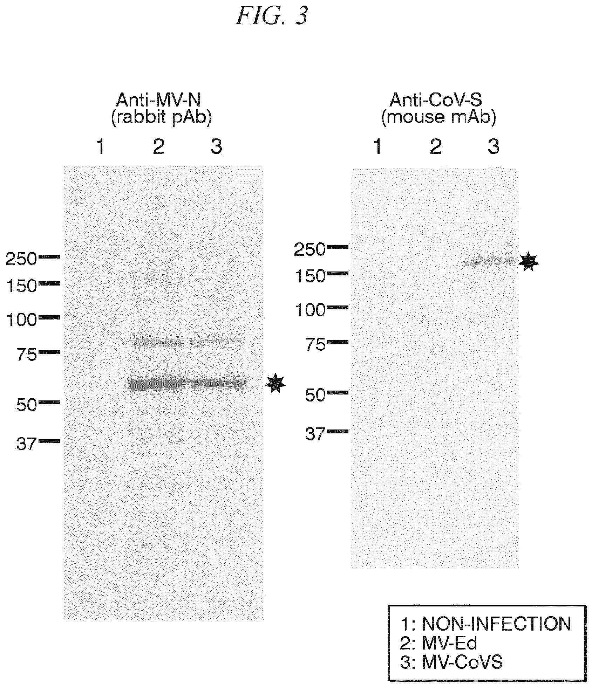

A Vero cell not infected with a measles virus (non-infected cell), a Vero cell infected with a measles virus MV-Ed (MV-Ed cell), and a Vero cell infected with a measles virus MV-CoVS (MV-CoVS cell) were subjected to Western blotting analysis using an antibody (anti-MV-N antibody) recognizing the N protein of the measles virus and an anti-CoV-S antibody. The results of Western blotting using the anti-MV-N antibody and the anti-rabbit IgG antibody are shown in FIG. 3 (left), and the results of Western blotting using the anti-CoV-S antibody and the anti-mouse IgG antibody are shown in FIG. 3 (right). A band of the MV-N protein was confirmed in both the MV-Ed cells and the MV-CoVS cells (FIG. 3 (left), asterisk), but a band of the CoV-S protein was confirmed only in the MV-CoVS cells (FIG. 3 (right), asterisk).

Furthermore, a hamster was inoculated with the measles virus MV-CoVS to investigate whether antibody production was induced. Specifically, the hamster was intraperitoneally (IP) inoculated twice with the measles virus MV-CoVS, and the blood was collected over time to measure the titer of the anti-CoV-S antibody in the serum. The second virus inoculation was performed at 5 weeks (on the 35th day) from the first inoculation. As a control, a hamster was inoculated with the measles virus MV-Ed in the same manner. The titer of the anti-CoV-S antibody in the serum was measured over time. The titer of the anti-CoV-S antibody in the serum was measured by an ELISA method using the serum.

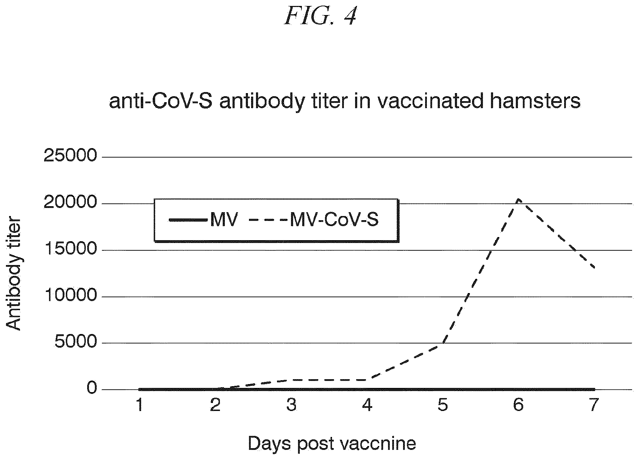

The results of measurement of the titer of the anti-CoV-S antibody in the serum over time from the first vaccination (day 0) are shown in FIG. 4. The production of the anti-CoV-S antibody in the serum was not observed in the hamster inoculated with the measles virus MV-Ed. In contrast, in the hamster inoculated twice with the measles virus MV-CoVS, the titer of the anti-CoV-S antibody in the serum was increased and the production of the anti-CoV-S antibody was induced by vaccinating twice. As shown in these results, the measles virus MV-CoVS has an immunity induction ability and is a useful candidate for a vaccine against COVID-19.

## Claims

1. A recombinant measles virus, comprising:
a gene encoding a protein of coronavirus SARS-CoV-2 inserted between an N gene region and a P gene region in a measles virus genome.

2. The recombinant measles virus according to Claim 1,
wherein the protein is a spike protein of SARS-CoV-2 or a partial protein thereof.

3. The recombinant measles virus according to Claim 1 of 2,
wherein the measles virus genome is an RNA consisting of a base sequence complementary to a base sequence set forth in SEQ ID NO: 1.

4. A genomic RNA of the recombinant measles virus according to any one of Claims 1 to 3.

5. An RNA consisting of a base sequence complementary to a base sequence set forth in SEQ ID NO: 3.

6. A DNA comprising:
a gene encoding a protein of SARS-CoV-2 inserted into a region ranging from a 1,686th base to a 1,694th base in a base sequence set forth in SEQ ID NO: 2.

7. A DNA comprising:
a gene encoding a protein of SARS-CoV-2 inserted into an Fse I recognition sequence located from a 1,686th base to a 1,694th base of a base sequence set forth in SEQ ID NO: 1.

8. A DNA consisting of a base sequence set forth in SEQ ID NO: 3.

9. A vector for producing a COVID-19 vaccine, the vector comprising:
the DNA according to any one of Claims 6 to 8.

10. A plasmid vector comprising:
a DNA consisting of a base sequence set forth in SEQ ID NO: 1.

11. A method for producing a recombinant measles virus, the method comprising:
transfecting a vector for producing a COVID-19 vaccine including the DNA according to any one of Claims 6 to 8 and an expression vector for an N protein, a P protein, and an L protein of a measles virus into a cell expressing an RNA polymerase capable of transcribing an RNA using the DNA as a template to produce a recombinant measles virus in the cell.

12. A pharmaceutical composition comprising:
the recombinant measles virus according to any one of Claims 1 to 3; and
a pharmaceutically acceptable carrier.

13. The pharmaceutical composition according to Claim 12,
wherein the pharmaceutical composition is used as a bivalent vaccine against measles and COVID-19.

14. A use of the recombinant measles virus according to any one of Claims 1 to 3 as a vaccine against COVID-19.

15. A use of the recombinant measles virus according to any one of Claims 1 to 3 for producing a vaccine against COVID-19.

16. A method for inducing an immune response against coronavirus SARS-CoV-2, the method comprising:
administering a pharmaceutical composition including the recombinant measles virus according to any one of Claims 1 to 3 to a subject in need of prevention of COVID-19.
